# EUROPEAN PATENT APPLICATION

(11) **EP 4 597 113 A1**
(43) Date of publication of application: **06.08.2025**
(21) Application number: 23873193.9
(22) Date of filing: 27.09.2023
(51) Int. Cl.: G01N 33/68, G01N 27/623

(54) **NOVEL INTERNAL STANDARD MATERIAL FOR MASS CALIBRATION**

(30) Priority: 28.09.2022 KR 20220123127
(71) Applicant: Seegene Medical Foundation, Seoul 04805 (KR)
(72) Inventor: CHUN, Jong Kee, Seoul 04805 (KR); BAEK, Je Hyun, Gwacheon-si, Gyeonggi-do 13824 (KR); YANG, Won Suk, Bucheon-si, Gyeonggi-do 14774 (KR); LEE, Saeyoung, Suwon-si, Gyeonggi-do 16361 (KR); JANG, Heejung, Seoul 02603 (KR); HWANG, Seohyun, Seoul 01755 (KR); PARK, Juri, Incheon 21422 (KR)
(74) Representative: Mewburn Ellis LLP
(86) International application number: PCT/KR2023/014999
(87) International publication number: WO 2024/072083

(57) **Abstract**

The present invention relates to an internal standard material for mass determination of a protein and a method for detecting a protein in a biological sample using the same. The present invention enables rapid calibration of the measured mass and quantitative value of the target protein with high confidence by a simple process of adding a recombinant protein in which a tag peptide is conjugated to the target protein into the sample as an internal standard. The present invention may be applied to highly sensitive analysis for the environmental samples and the diagnosis of infectious pathogens by clearly discriminating the target protein of high molecular weight from other proteins in the sample with small mass differences, even using low resolution mass spectrometry instruments.

## Description

### Technical Field

The present invention relates to a method for accurately and efficiently correcting the measured mass value of an analyte protein by using as an internal standard a recombinant protein in which a specific peptide whose mass information is known is conjugated to the same protein as the analyte protein.

### Background Art

Matrix Desorption/ Ionization Time of Flight (MALDI-TOF) mass spectrometry is an instrument that measures the molecular weight of the analyte by drying a mixture of sample and matrix to form a crystal structure, desorbing and ionizing it by irradiation with a laser, and measuring the time of flight to the detector. Maldi-TOF has the advantage of rapid mass spectrometry of macromolecules such as proteins because it does not fragment the analyte, but it also has the limitation of low-resolution, making it difficult to accurately identify proteins with small mass differences. It is also noted that drying the mixture of sample and matrix produces an inhomogeneous crystal structure, resulting in low reproducibility.

Currently, mass calibration using external standards is commonly applied to improve the accuracy of mass spectrometry by MALDI-TOF, but this method involves measuring the standard at one or more spots to obtain a calibration parameter, which is then used to calibrate the sample at another spot. When calibrating mass values of high molecular proteins of 20 kDa or more using external standards in low-resolution MALDI-TOF instruments, it is difficult to expect accurate mass calibration due to the wide error range of mass values depending on protein size, and there are limitations in separating/identifying proteins of similar size. In addition, while the mass spectrometer-based internal standards have been reported, most of them are mainly used in peptide-level quantification methods, and the development for protein-level quantification methods is also a challenge. Therefore, it is necessary to develop efficient mass calibration and quantitative calibration methods that enable more accurate mass analysis at the protein level.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to develop an efficient mass spectrometry method for the accurate and rapid separation and identification of a high molecular weight target protein in a sample using low resolution mass spectrometry equipment. As a result, the present inventors have found that when a recombinant protein with a Tag peptide of any amino acid sequence whose molecular weight is already known is conjugated to the N- or C-terminus of a protein with the same amino acid sequence as the target protein is used as an internal standard, the mass and quantitative value of the target protein may be calibrated simply and accurately based on the molecular weight of the Tag peptide corresponding to the difference in molecular weight between the target protein and the internal standard, thereby completing the present invention.

Accordingly, it is an object of the present invention to provide an internal standard material for the mass determination of a protein.

It is another object of the present invention to provide a method for detecting a protein in a biological sample using the internal standard material.

Other objects and advantages of the present invention will become more apparent from the following detailed description, the appended claims, and the accompanying drawings.

### Technical Solution

In one aspect of this invention, there is provided an internal standard material for mass determination of a protein, comprising:
(a) the protein to be analyzed; and
(b) a tag peptide comprising 1 to 100 consecutive random amino acids bound to the N-terminus or C-terminus of the protein to be analyzed.

The present inventors have made intensive studies to develop an efficient mass spectrometry method for the accurate and rapid separation and identification of a high molecular weight target protein in a sample using low resolution mass spectrometry equipment. As a result, the present inventors have found that when a recombinant protein with a Tag peptide of any amino acid sequence whose molecular weight is already known is conjugated to the N- or C-terminus of a protein with the same amino acid sequence as the target protein is used as an internal standard, the mass and quantitative value of the target protein may be calibrated simply and accurately based on the molecular weight of the Tag peptide corresponding to the difference in molecular weight between the target protein and the internal standard.

As used herein, the term "protein" refers to a linear molecule formed by amino acid residues linked together by peptide bonds. In the present invention, the proteins to be detected by mass spectrometry may be proteins that are biological markers, for example, for the presence of a pathogenic strain in a sample (i.e., diagnosis of pathogen infection), or identification of its species and phenotype.

As used herein, the term "pathogenic strain" refers to any bacteria that act as the cause of an infection or disease, including, for example, but not limited to, *Staphylococcus aureus, Streptococcus, Escherichia coli, Klebsiella pneumoniae, Pseudomonas aeruginosa, Pseudomonas otitidis, Micrococcus luteus, Citrobacter koseri, Proteus mirabilis,* and *Mycobacterium ulcerans.*

The target proteins to be analyzed in the present invention include, for example, marker proteins that can predict the presence of a pathogenic strain as well as the phenotype thereof, such as antibiotic resistance.

As used herein, the expression "having resistance to antibiotics" means that a specific pathogenic microorganism can grow even in an environment in which antibiotics against the microorganism are present at high concentration or in an effective amount. Whether the pathogenic microorganism has antibiotic resistance may be determined by detecting the presence of an enzyme protein, which is secreted by the pathogenic microorganism and removes or reduces the activities of the antibiotics by degrading the antibiotics. For example, beta-lactam antibiotics that inhibit bacterial cell wall synthesis, such as penicillin, cephalosporin, monobactam, and carbapenem, are inactivated by β-lactamase so that they cannot inhibit pathogens expressing β-lactamase. Accordingly, the term "resistance" is used interchangeably with the term "low therapeutic responsiveness".

As used herein, the term "internal standard material" refers to a compound with a known concentration or mass that is added in an indicated amount to the sample to be analyzed. It is used to correct the quantitative error in the mass of target analyte (i.e., the target protein) by comparing the signal from the target analyte with the signal from the internal reference material and assessing their difference or ratio.

As used herein, the term "tag peptide" refers to an amino acid sequence genetically transferred into a recombinant protein for various purposes, such as facilitating the isolation, purification, solubilization, visual labeling, etc. of a target protein. The tag peptide of the present invention may be used, as a component of an internal standard, to correct the mass value of the target protein by being conjugated to the N-terminus or C-terminus of a recombinant protein having the same amino acid sequence as the target protein to be detected, so that a mass difference equal to the molecular weight of the tag peptide occurs between the target protein and the internal standard material during mass spectrometry. Accordingly, the tag peptide of the present invention does not need to be removed by a protease or the like after analysis, as it only constitutes a part of the internal standard material, unlike in the case of binding directly to the target protein to be analyzed.

According to a specific embodiment of the invention, the tag peptide comprises 2 to 80 consecutive random amino acids bound to the C-terminus of the protein to be analyzed. More specifically, the tag peptide comprises 2 to 70 consecutive random amino acids bound to the C-terminus, more specifically, the tag peptide comprises 2 to 60 consecutive random amino acids bound to the C-terminus, more specifically, the tag peptide comprises 2 to 50 consecutive random amino acids bound to the C-terminus, more specifically, the tag peptide comprises 2 to 40 consecutive random amino acids bound to the C-terminus, more specifically, the tag peptide comprises 2 to 30 consecutive random amino acids bound to the C-terminus, more specifically, the tag peptide comprises 2 to 20 consecutive random amino acids bound to the C-terminus, more specifically, the tag peptide comprises 2 to 10 consecutive random amino acids bound to the C-terminus, and most specifically, the tag peptide comprises 2 to 8 consecutive random amino acids bound to the C-terminus.

According to a specific embodiment, the tag peptide is selected from the group consisting of a repeat sequence of the same amino acid residues; a consecutive sequence of different amino acid residues; a repeat of a consecutive sequence of different amino acid residues; and combinations thereof.

In a more specific embodiment, the same amino acid is histidine (His).

According to a more specific embodiment, the consecutive sequence of different amino acid residues comprises an amino acid of SEQ ID NO: 7. According to the present invention, SEQ ID NO: 7 is an amino acid sequence of a Strep-tag peptide (Trp-Ser-His-Pro-Gln-Phe-Glu-Lys) having an affinity for streptavidin.

The tag peptide of the present invention is not limited to the examples described above, but can be any peptide that results in a molecular weight difference between the target protein and the internal standard material by a mass value of the tag peptide calculated based on known amino acid sequence thereof.

According to a specific embodiment, the mass spectrometry is selected from the group consisting of matrix-assisted laser desorption/ ionization time-of-flight (MALDI-TOF) mass spectrometry, surface enhanced laser desorption/ ionization time-of-flight (SELDI-TOF) mass spectrometry, electrospray ionisation time-of-flight (ESI-TOF) mass spectrometry, liquid chromatography-mass spectrometry (LC-MS) and liquid chromatography-mass spectrometry/ mass spectrometry (LC-MS/MS). More specifically, the mass spectrometry is matrix desorption/ ionization time-of-flight (MALDI-TOF) mass spectrometry.

MALDI-TOF mass spectrometry is a method in which a sample supported by a matrix is desorbed and ionized by irradiation with a laser, and then the molecular weights of the generated ions are analyzed by measuring the time (Time-of-Flight) taken for the ions to reach a detector. According to this method, it is possible to quickly and accurately measure the mass of a large biomolecule such as a protein, because fragmentation of the target material does not occur. When the ionized molecule is accelerated by an electric field and the flight time is measured, a mass-to-charge ratio (m/z) is generated, and the molecular weight of the target material may be determined through this m/z value.

Currently, to improve the accuracy of mass spectrometry by MALDI-TOF, mass correction is mainly performed using external standards. However, for high molecular weight proteins of 20 kDa or more, measurement with low-resolution MALDI-TOF instruments using external standards causes too large errors in the mass value for reliable analysis. By using a recombinant protein with a tag peptide conjugated to the target protein as an internal standard material, the present invention may clearly identify the high molecular weight target protein from other proteins with small mass differences in the sample even using low resolution MALDI-TOF equipment.

In another aspect of this invention, there is provided a nucleic acid molecule encoding the internal standard material of the invention.

As used herein, the term "nucleic acid molecule" is meant to encompass DNA (gDNA and cDNA) and RNA molecules. Nucleotides, which are the basic structural units in nucleic acid molecules, include not only natural nucleotides, but also analogues having modified sugar or base moieties (Scheit, Nucleotide Analogs, John Wiley, New York (1980); Uhlman and Peyman, Chemical Reviews, 90:543-584 (1990)). It will be clear to those skilled in the art that the nucleotide sequence encoding the amino acid sequence of an internal standard material of the present invention, i.e., a recombinant protein with a tag peptide bound to the protein of interest, is not limited to the nucleotide sequences listed in the appended sequence list or directly cloned in the Examples of the present invention. The modifications of nucleotides included in the Examples encompass modifications that do not result in protein-level changes, i.e., nucleic acid molecules having functionally equivalent codons, codons that code for the same amino acid due to codon degeneracy, or codons that code for biologically equivalent amino acids.

Considering the above-described variations having biologically equivalent activity, it is interpreted that the nucleotides set forth in the Sequence Listing or directly cloned in the Examples, as well as sequences that exhibit substantial identity to the sequences. Substantial identity means a sequence showing at least 70% homology, specifically at least 75% homology, more specifically at least 80% homology, more specifically at least 85 homology, more specifically at least 90% homology, most specifically at least 95% homology as determined by aligning the sequence of the present invention with any other sequence to correspond to each other as much as possible and analyzing the aligned sequence using an algorithm commonly used in the art. Alignment methods for sequence comparison are disclosed in Huang et al. Comp. Appl. BioSci. 8:155-65 (1992) and Pearson et al. Meth. Mol. Biol. 24:307-31(1994).

According to one embodiment, the internal standard material of the present invention may be obtained recombinantly by expressing a nucleic acid molecule encoding it in a host cell.

As used herein, the term "express" refers to being artificially replicated as an extrachromosomal factor or by chromosomal integration in a target cell via a gene delivery system to cause the target cell to express an exogenous gene or overexpress an endogenous gene. Accordingly, "express" may be used interchangeably with "transformation", "transfection", or "transduction". More specifically, "to express" in the present invention refers to cause a target cell to artificially express an exogenous gene.

The term "gene delivery system" or "gene delivery vehicle" as used herein refers to any means for delivering a gene into a cell, and the term "gene delivery" has the same meaning as intracellular transduction of the gene. At the cell or tissue level, gene delivery has the same meaning as spread of the gene. Thus, the gene delivery system of the present invention may be referred to as a gene transduction system or a gene spread system.

To construct the gene delivery system of the present invention, the nucleotide sequence is operatively linked to a suitable expression regulatory sequence within a suitable expression construct. As used herein, the term "operatively linked" refers to a functional linkage between a nucleic acid expression regulatory sequence (e.g., a promoter, a signal sequence, or an array of transcription regulation factor binding sites) and the target nucleic acid sequence. Through the linkage, the regulatory sequence regulates the transcription and/or translation of the target nucleic acid sequence.

The gene delivery system of the present invention can be produced in a variety of forms, including (i) naked recombinant DNA molecules, (ii) plasmids, (iii) viral vectors, and (iv) liposomes or niosomes containing the naked recombinant DNA molecules or plasmids.

In still another aspect of this invention, there is provided a method for detecting a protein in a biological sample comprising:
(a) adding the internal standard material of any one of claims 1 to 7 to the biological sample comprising the protein to be analyzed;
(b) measuring a mass value of the protein to be analyzed and the internal standard material in the biological sample; and
(c) calibrating the measured mass value of the protein to be analyzed based on the measured mass value of the internal standard material.

As used herein, the term "biological sample" refers to any material likely to contain the target protein to be analyzed or cells expressing them, cultures thereof, including samples isolated from living organisms (e.g., blood, plasma, serum, saliva, tissues, organs, etc.), materials taken from the environment (e.g., water, air, soil, etc.), or artificially mixed samples.

According to one embodiment, the step (b) is performed using a mass spectrometry method selected from the group consisting of MALDI-TOF mass spectrometry, SELDI-TOF mass spectrometry, ESI-TOF mass spectrometry, LC-MS and LC-MS/MS. More specifically, the step (b) is performed using MALDI-TOF mass spectrometry.

The mass spectrometry methods utilized in the present invention have already been described above in detail and are therefore omitted to avoid undue redundancy.

In the method of the present invention, the internal standard is added to the biological sample to be analyzed, followed by protein separation and purification, and mass spectrometry analysis. If a protein with a molecular weight difference corresponding to the mass of the tag peptide relative to the internal standard material is detected, it can be determined that the target protein is present in the sample. By calibrating the mass value of the target protein based on the mass of the internal standard material, the reproducibility of each mass measurement is improved, bringing it closer to the theoretical mass value, thereby significantly enhancing the reliability of mass measurement

In still another aspect of this invention, there is provided an internal standard material for quantitation of a protein, comprising:
(a) the protein to be analyzed; and
(b) a tag peptide comprising 1 to 100 consecutive random amino acids bound to the N-terminus or C-terminus of the protein to be analyzed.

In still another aspect of this invention, there is provided a method for quantitation of a protein in a biological sample comprising:
(a) adding the internal standard material of claim 12 to the biological sample comprising the protein to be analyzed;
(b) measuring a mass value of the protein to be analyzed and the internal standard material in the biological sample; and
(c) calibrating a quantitative value of the protein to be analyzed based on the signal intensity of the measured mass value of the internal standard material.

The internal standard material of the present invention where the tag peptide conjugated to the end of the protein having the same amino acid sequence as the protein to be analyzed, has already been described above in detail and are therefore omitted to avoid undue redundancy.

In the method of the present invention, the internal standard material is added to the biological sample to be analyzed, followed by protein separation, purification, and mass spectrometry analysis. By calibrating the target protein intensity value based on the peak intensity value of the mass spectrum which objectively reflects the concentration of the internal standard material in the sample, the method can significantly improve the reproducibility and reliability not only for the target protein's mass measurement but also for its quantification.

In still another aspect of this invention, there is provided an internal standard material for mass determination of a protein comprising:
(a) any protein whose mass value is known; and
(b) a tag peptide comprising 1 to 100 consecutive random amino acids bound to the N-terminus or C-terminus of the protein.

In still another aspect of this invention, there is provided a method for detecting a protein in a biological sample comprising:
(a) adding the internal standard material of claim 14 to the biological sample comprising the protein to be analyzed;
(b) measuring a mass value of the protein to be analyzed and the internal standard material in the biological sample; and
(c) calibrating the measured mass value of the protein to be analyzed based on the measured mass value of the internal standard material.

In still another aspect of this invention, there is provided an internal standard material for quantitation of a protein comprising:
(a) any protein whose mass value is known; and
(b) a tag peptide comprising 1 to 100 consecutive random amino acids bound to the N-terminus or C-terminus of the protein.

In still another aspect of this invention, there is provided a method for quantitation of a protein in a biological sample comprising:
(a) adding the internal standard material of claim 17 to the biological sample comprising the protein to be analyzed;
(b) measuring a mass value of the protein to be analyzed and the internal standard material in the biological sample; and
(c) calibrating a quantitative value of the protein to be analyzed based on the signal intensity of the measured mass value of the internal standard material.

In still another aspect of this invention, there is provided a nucleic acid molecule encoding the internal standard material of the invention.

The tag peptides, nucleic acid molecules, mass spectrometry methods and methods for detection and quantification of proteins to be analyzed have already been described above in detail and are therefore omitted to avoid undue redundancy.

According to the present invention, the internal standard material of the invention is not limited to a recombinant protein in which a tag peptide is conjugated to a protein having the same amino acid sequence as the target protein to be analyzed. It may also include a recombinant protein in which a tag peptide is conjugated to an arbitrary protein that is different from the target protein but has known mass value information, allowing for the obvious derivation of the mass difference with the target protein. In this case, the molecular weight difference between the target protein and the internal standard material is the sum of the molecular weight difference between the two proteins and the molecular weight of the tag peptide. Through this approach, it is possible to simultaneously detect or quantify multiple target proteins with various mass values within a sample.

According to a specific embodiment, any of the (arbitrary) protein of the invention has a mass value difference of ± 5,000 Da or less from the target protein to be analyzed, more specifically ± 4,000 Da or less, even more specifically ± 3,000 Da or less, and most specifically ± 2,000 Da or less.

### Advantageous Effects

The features and advantages of the present invention are summarized as follows:
(a) The present invention provides an internal standard material for mass determination of a protein and a method for detecting a protein in a biological sample using the same.
(b) The present invention enables rapid calibration of the measured mass and quantitative value of the target protein with high confidence by a simple process of adding a recombinant protein in which a tag peptide is conjugated to the target protein into the sample as an internal standard.
(c) The present invention may be applied to highly sensitive analysis for the environmental samples and the diagnosis of infectious pathogens by clearly discriminating the target protein of high molecular weight from other proteins in the sample with small mass differences, even using low resolution mass spectrometry instruments.

### Brief Description of Drawings

FIG. 1 shows the results of SDS-PAGE analysis confirming the expression and size of each tagged protein (KPC-2_6xhis-tag, KPC-2_2xhis-tag, KPC-2_strep-tag).
FIG. 2 represents the isolation and purification results of a tagged protein (KPC-2_6xhis-tag) using chromatography. FIG. 2a shows the results of metal affinity chromatography and FIG. 2b shows the results of separation and purification using an anion exchange resin column.
FIG. 3 shows the mass spectrum of a tagged protein (KPC-2_6xhis-tag) obtained by bottom-up mass spectrometry on a high-resolution mass spectrometer.
FIG. 4 shows the results of MALDI-TOF analysis using KPC-2_6xhis-tag as an internal standard material and mass calibration based on the difference in mass values between the target protein and the internal standard material.
FIG. 5 shows the results of MALDI-TOF analysis using KPC-2_2xhis-tag as an internal standard material and mass calibration based on the difference in mass values between the target protein and the internal material.
FIG. 6 shows the results of MALDI-TOF analysis using KPC-2_strep-tag as an internal standard material and mass calibration based on the difference in mass values between the target protein and the internal standard material.
FIG. 7 shows the MALDI-TOF analysis results for different concentrations of the tagged protein (KPC-2_6xHis-tag) and the corresponding mass calibration effects.
FIG. 8 shows the MALDI-TOF analysis results for different concentrations of the tagged protein (KPC-2_6xHis-tag) and the corresponding quantitative calibration effects.
FIG. 9 presents the results of target protein identification using the tagged protein in 43 clinical strains. The mass differences of the target protein were compared under three conditions: without applying the tagged protein as an internal standard material, with its application, and after mass calibration following its application.

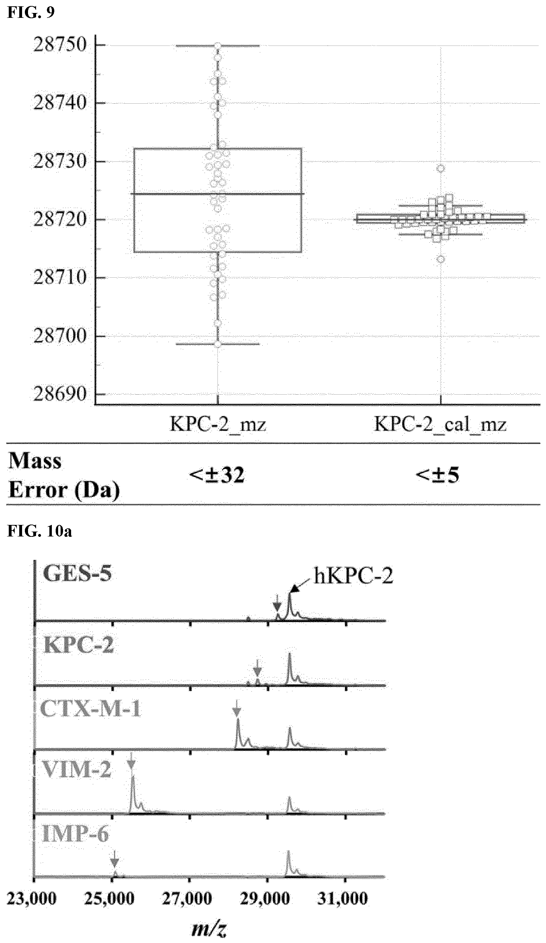

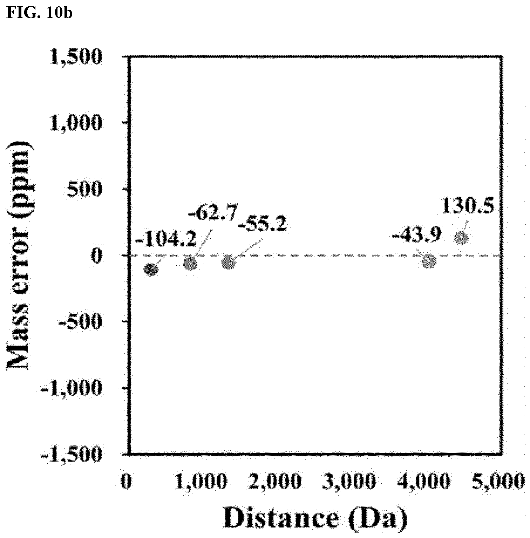

FIG. 10 is an illustration of the mass error according to the distance from the target pathogenic protein when hKPC-2 is applied as an internal standard material. FIG. 10a is a mass spectrum of the target proteins showing the distance between each target protein and the internal standard material (hKPC-2). Each arrow indicates the peak of the respective pathogenic protein. FIG. 10b is a graph showing the mass error of the target proteins against the molecular weight distance between the internal standard material (hKPC-2) and the target proteins. All data are expressed as standard deviation (n = 3).
FIG. 11 illustrates the mass error of KPC-type proteins derived using an internal standard material (KPC-2). FIG. 11a shows the mass spectra of KPC-type proteins after calibration with an external standard (Ex-Cal, KPC-2) and an internal standard (In-Cal, hKPC-2) materials. FIG. 11b is a graph showing the average mass error of each protein for Ex-Cal (KPC-2) and In-Cal (hKPC-2) as a standard deviation (n=3).

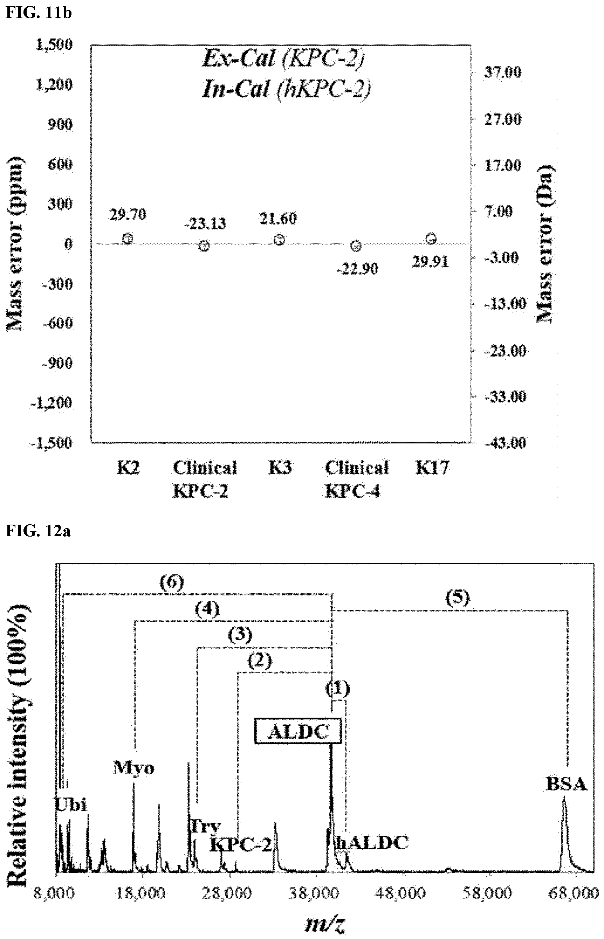

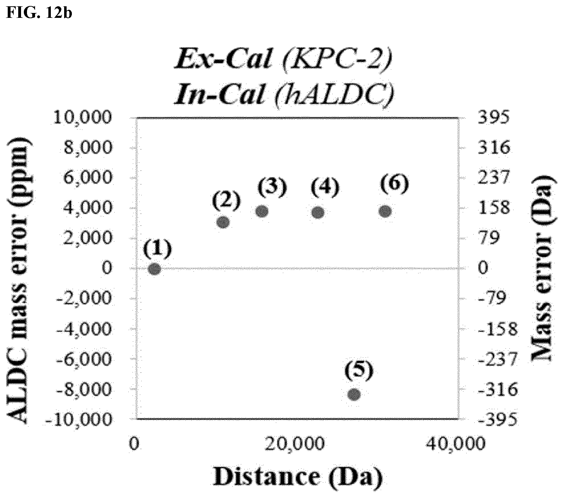

FIG. 12 is an illustration of the mass error of ADLC according to the distance from the internal standard after mass calibration. FIG. 12a is an example of a mass spectrum showing mass distance from an internal standard material. (1) to (6) represent the mass distance between each internal standard [(1) nhALDC, (2) KPC-2, (3) trypsinogen, (4) myoglobin, (5) BSA, and (6) ubiquitin] and the target protein ALDC. The theoretical mass differences (ΔM) with the single-charged ALDC ion are (1) 2119.7, (2) 10758.1, (3) 15495.2, (4) 22524.9 (5) 26920.9, and (6) 30911.4 Da, respectively. FIG. 12b shows the mass error of ALDC measured by Ex-Cal (KPC-2) and In-Cal (h).

### Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

### Examples

### Cloning the tagged genes and preparation of the strain.

Based on the target gene sequence information, the desired tag was labeled and synthesized.

The following primers were constructed to synthesize genes coding for a KPC-2 protein tagged with six histidine repeats at the C-terminus; a KPC-2 protein tagged with two histidine repeats at the C-terminus; and a KPC-2 protein tagged with two Strep repeats at the C-terminus:

### 1) C-terminal His6 tag KPC-2 (KPC-2_6xhis-tag)

Primer 1: 5'-AACTGCAGGATGTCACTGTATCGCCGTCTA-3' (30mer)
Primer 2: 5'-GGAATTCTCAGTGGTGGTGGTGGTGGTGGTGCTGCCCGTTGACGCCCA-3' (45mer)

### 2) C-terminal His2 tag KPC-2 (KPC-2_2xhis-tag)

Primer 1: 5'-AACTGCAGGATGTCACTGTATCGCCGTCTA-3' (30mer)
Primer 2: 5'-GGAATTCTCAGTGGTGCTGCCCGTTGACGCCCA-3' (33mer)

### 3) C-terminal Strep2 tag KPC-2

Primer 1: 5'-AACTGCAGGATGTCACTGTATCGCCGTCTA-3' (30mer)
Primer 2: 5'-GGAATTCTCATTTTTCGAACTGCGGGTGGCTCCACTGCCCGTTGACGCCCA-3' (51mer)

The primers were designed with restriction enzyme sites for cloning, and ORF (Open Reading Frame) aligned to drive direct expression from the cloning vector. For PCR, the reaction solution was prepared using 1 µl of template DNA, 1.25 µl of 5' primer, 1.25 µl of 3' primer, 1 µl of dNTPs, 10 µl of 5X buffer, and 5X GC enhancer buffer for a total of 50 µl PCR reaction. PCR was performed under the following conditions:
1) Denaturation, - 98°C for 10 sec;
2) Annealing, - 57 at°C for 30 sec;
3) Extension, - 72°C for 30 sec.

Cloning for the construction of recombinant expression vectors was performed as follows:
1) The insert gene and vector were cut into sticky ends of DNA using restriction enzymes; 2) The insert gene was ligated into the vector using DNA ligation enzyme; 3) The vector was transformed into *E. coli* top10; 4) The recombinant *E. coli* was screened by white/blue screening method. Then, 5) the recombinant plasmid was extracted from the selected strains; and 6) the insertion gene was finally confirmed by DNA sequencing.

### Expression and identification of tagged proteins

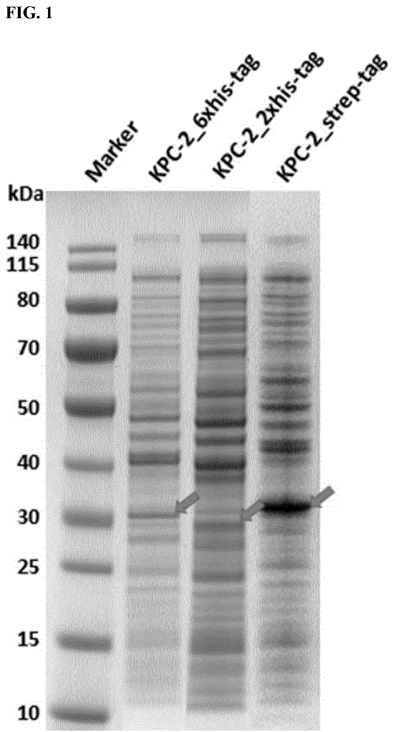

Transformed *E. coli* were inoculated into Luria-bertani (LB) liquid medium containing 50 mg/L ampicillin, 37°C and incubated for 16 hours. To determine the expression and size of the tag-labeled protein, the cultures were centrifuged at 4,000 rpm for 15 minutes, and the supernatant was removed to harvest the cells. The harvested cells were added to SDS buffer, heated at 95°C for 10 min, and centrifuged at 14,000 g for 10 min. The prepared samples were subjected to SDS-PAGE gel analysis to confirm the expression and size of tagged proteins (FIG. 1).

### Preparation of sample

### (1) Sample preparation using sonication

The cultures of the expressed strains were centrifuged at 4,000 rpm for 15 min, and the supernatant was removed to harvest the cells. The harvested cells were resuspended by adding 500 mM NaCl, 25 mM Tris-HCl, pH8.0. The cells were disrupted using an Ultrasonic Processor (VC-505, Sonics & Materials, USA). The cells were then centrifuged at 14,000 g for 10 min at 4°C to separate the supernatant (hereafter, crude enzyme solution) and precipitate, and the crude enzyme solution was recovered for isolation/purification of tag-labeled proteins. Ultrasonication can be performed using either probe-type or sonic baths, and the method described above is an example of pretreatment using probe-type ultrasonication.

### (2) Sample preparation using osmotic lysis methods

Sample preparation was performed in the following steps: 1) Cells were harvested by centrifuging the expressed strain cultures and removing the supernatant. 2) Harvested cells were resuspended by adding hypertonic solution (500 mM NaCl, 25 mM Tris-HCl, pH8.0) and allowed to react for 10 min at room temperature. 3) The supernatant was removed by centrifugation at 14,000g for 10 min at 4°C, resuspended by adding tertiary distilled water, and reacted for 10 min at room temperature. 4) After centrifugation at 14,000 g for 10 min at 4°C, the crude enzyme solution was recovered.

### (3) Sample Preparation with Surfactant

Pretreatment methods were as follows: 1) Cells were harvested by centrifuging the expressed strain cultures and removing the supernatant. 2) BugBuster reagent was added to the harvested cells for resuspension and then reacted for about 20 minutes at room temperature. 3) After centrifugation at 14,000 g for 10 min at 4°C, the crude enzyme solution was recovered.

### Isolation and purification of tagged proteins

Metal affinity chromatography and ion exchange chromatography were performed for the separation/purification of tag-labeled proteins. Ni-NTA resin was used for the metal affinity chromatography, a column with Q-resin was used for the anion exchange chromatography.

### (1) Anion exchange resin chromatography

The column containing Q-resin was loaded with the crude enzyme solution and the eluted solution was collected. The column was washed with 20 mM tris-HCl, pH8.0 buffer, and elution buffers containing 200 mM NaCl, 400 mM NaCl, and 600 mM NaCl were loaded sequentially and the eluate was collected in each compartment.

### (2) Metal affinity chromatography

The crude enzyme solution was loaded onto a column containing Ni-NTA resin and the eluting solution was collected. The column was washed with 20 mM tris-HCl, pH8.0 buffer, and elution buffers containing 50 mM imidazole, 100 mM imidazole, 300 mM imidazole, and 500 mM imidazole were loaded sequentially and the eluate was collected in each compartment.

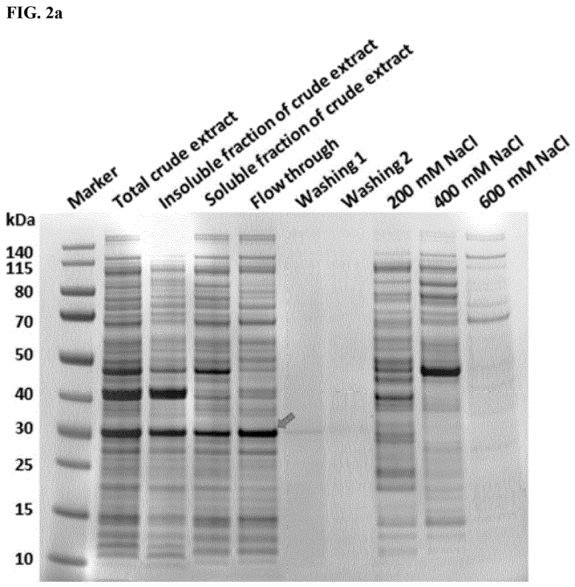

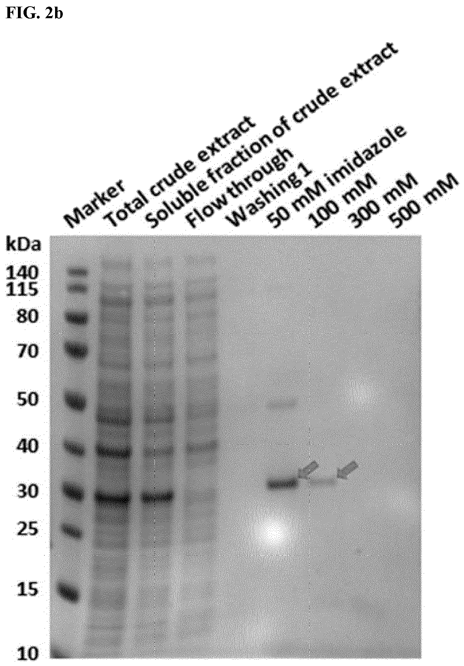

Finally, above two chromatographies were performed sequentially to isolate/ purify the tagged protein (FIG. 2).

### Desalting purified proteins and buffer exchange

The concentrating filter was washed with water at 3500 rpm for 5 min, and the remaining solution was collected by loading the purified protein and water onto the concentrating filter at 3500 rpm for 5 min. The same method was repeated four times to obtain the protein by desalting and buffer exchange.

### Identification of tagged proteins

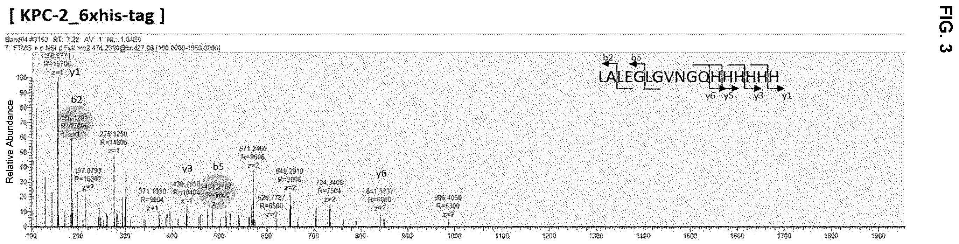

The tagged proteins of which the expression and size were confirmed by SDS-PAGE were confirmed using an in-gel digestion method (bottom-up method) and LC-MS/MS method (FIG. 3).

### (1) In-gel digestion

Only bands corresponding to tagged proteins were taken from the SDS-PAGE gel and then destained. The decolorized gel was subjected to reduction/alkylation, cleaved using trypsin enzyme, and the cleaved peptides were recovered by desalting using a C18 tip.

### (2) LC-MS/MS

To determine the sequence and range of tagged proteins expressed within the strains, Evosep liquid chromatography and high-resolution mass spectrometry (Q-Exactive HF-X mass spectrometer system) were performed. Desalted peptide samples were dissolved in 0.1% formic acid solution, and the sample was loaded onto the column. Peptide samples were separated using a C18 column (8 cm x 100 µm, 3 µm: EV1064) and nano-flow liquid chromatography. The EVOSEP SPD100 method (11.5 min) was used.
- Buffer A: 0.1% formic acid in water/Buffer B: 0.1% formic acid in acetonitrile.

The mass spectrometer parameters used in this case were
- Resolution: Full MS 60,000, applied with MS2 15,000
- Full MS: 300 to 2,000 m/z, 100 msec
- MS2: 28 msec, NCE 27, 1, >6 gas ionizers are not subject to MS2

Thermo's Proteome Discoverer (v2.4) search engine was used as software for peptide and protein identification with bottom-up data. Protein/peptide identifications were performed based on 1% FDR. From analyzed data, six additional histidine amino acids bound to the C-terminus of the target protein were identified by comparing theoretical mass values of the precursor and fragment ions.

### Mass spectrometry of target and tag-labeled proteins using MALDI-TOF

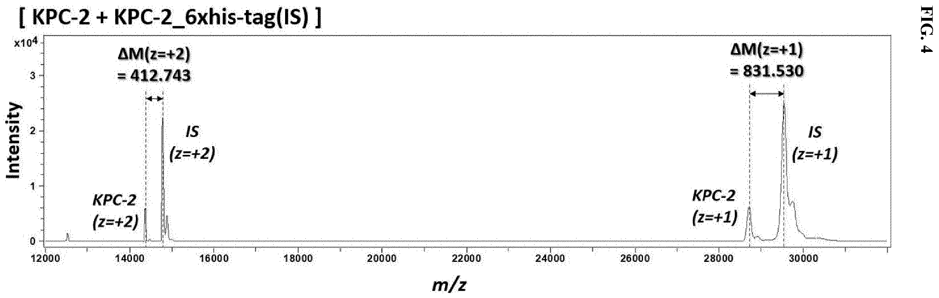

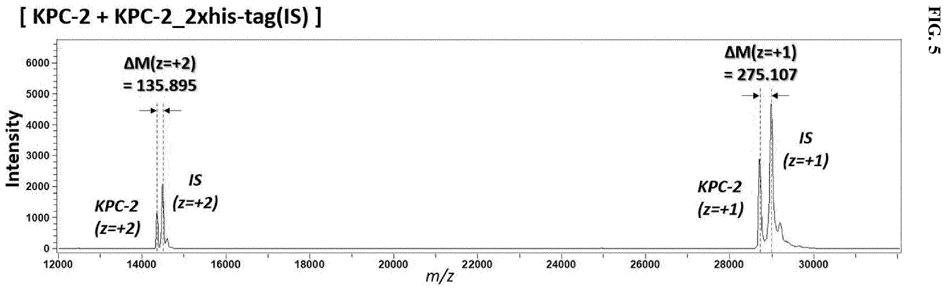

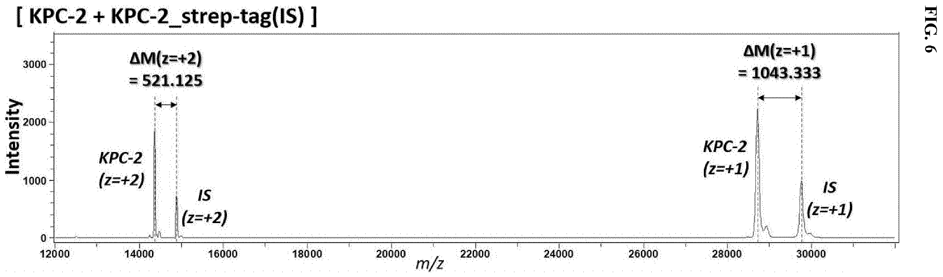

To confirm the mass values and peak intensities of the target protein and tagged protein, MALDI-TOF was performed on a mixture of the target protein and tagged protein (FIGs. 4-6). Bruker Biotyper MALDI-TOF MS instrument was used, and 1 µL of the mixed solution of target protein and tagged protein and 1 µL of sinapinic acid (SA) substrate (20 mg/mL in 0.1% TFA/50% acetonitrile) were placed on the plate spot, mixed, and thoroughly dried before use.

The pulse ion extraction time was 450 ns, and random position acquisition was performed with a total of 2,000 laser shots, applying 40 shots per position. The spectral data were accumulated to obtain the final results.

Mass spectra were obtained in cation and linear mode with a laser frequency of 100 Hz over the 12,000 to 32,000 m/z range, with simultaneous detection of +1 and +2 ions.

Mass spectral data were acquired using Bruker's Flex Control version 4.1 software and processed by Flexa Analysis version 4.1 software with smoothing using the Savitzky Golay method and baseline subtraction using the TopHat method. The intensity of each peak were measured using the Centroid peak detection algorithm.

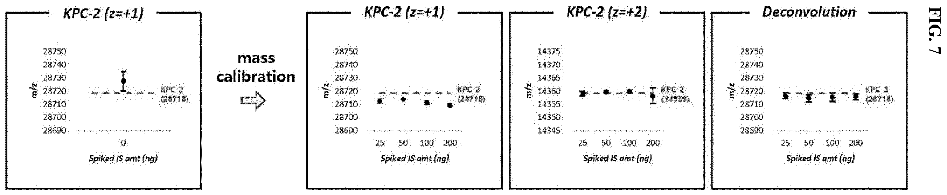

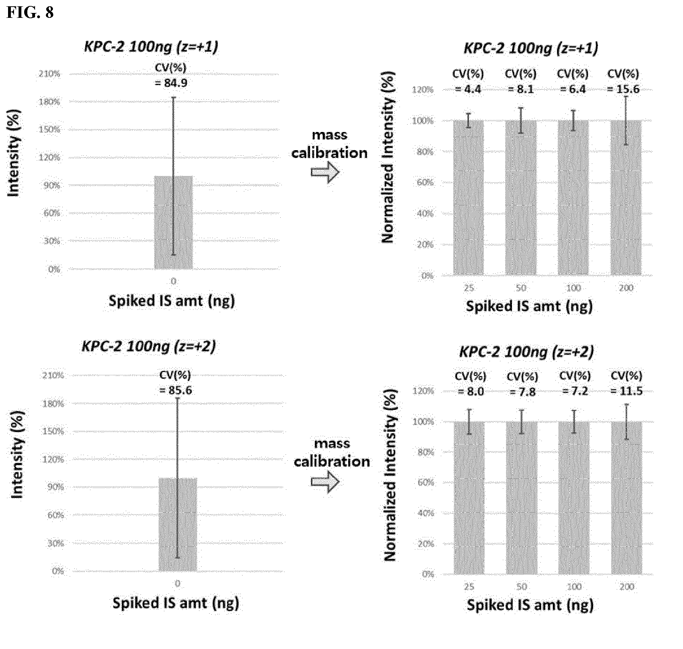

Calibration of the target protein mass value based on the mass value of the internal standard material, tagged protein, showed in all concentration combinations, an improvement in mass reproducibility, with mass values approaching the theoretical mass (FIG.7). In addition, the calibration of the target protein intensity based on the intensity of the tagged protein also showed a significant improvement in the reproducibility of the target protein intensity (FIG. 8).

### Mass calibration of clinical strain-derived target proteins with tagged proteins

To validate the mass calibration results of the target protein (KPC) derived from clinical strains using the tagged protein of the present invention, clinical strains genotyped as KPC were cultured on blood agar plate media. The target protein was identified in the same manner as described above for the cultured clinical strains. Specifically, a tag-labeled protein (MW=29,541Da) was used as an internal standard material to calibrate the mass value of the mass spectrum containing the KPC target protein (MW=28,718 Da), and the target protein was identified from a total of 43 clinical strains using the calibrated mass value (FIG. 9).

### Determining Calibrated Mass Ranges with IS Proteins

To evaluate the appropriate mass range of internal standards material (tagged protein), the present inventors determined the mass error values of three carbapenemases (IMP-6, VIM-2, and GES-5) and one beta-lactamase (CTX-M-1) whose masses differed from the internal standards by several hundred to 5000 Da. As a result, as shown in FIG. 10, all proteins exhibited mass errors of less than 3.2 Da. Among them, CTX-M-1 and VIM-2 have very low errors (<1.6 Da) compared to other proteins, and their distance from the internal standard material is 1331 and 4026 Da, respectively. IMP-6 had the largest mass difference from the internal standard, hKPC-2, and the largest mass error of all proteins (FIG. 10). The average mass error for all proteins derived using the external (Ex-Cal, KPC-2) and internal (In-Cal, hKPC-2) standards was ~83.5 ppm, with a distribution of mass errors ranging from - 3.0 to +3.2 Da.

### Identification of mass-calibrated KPC subtypes using hKPC-2

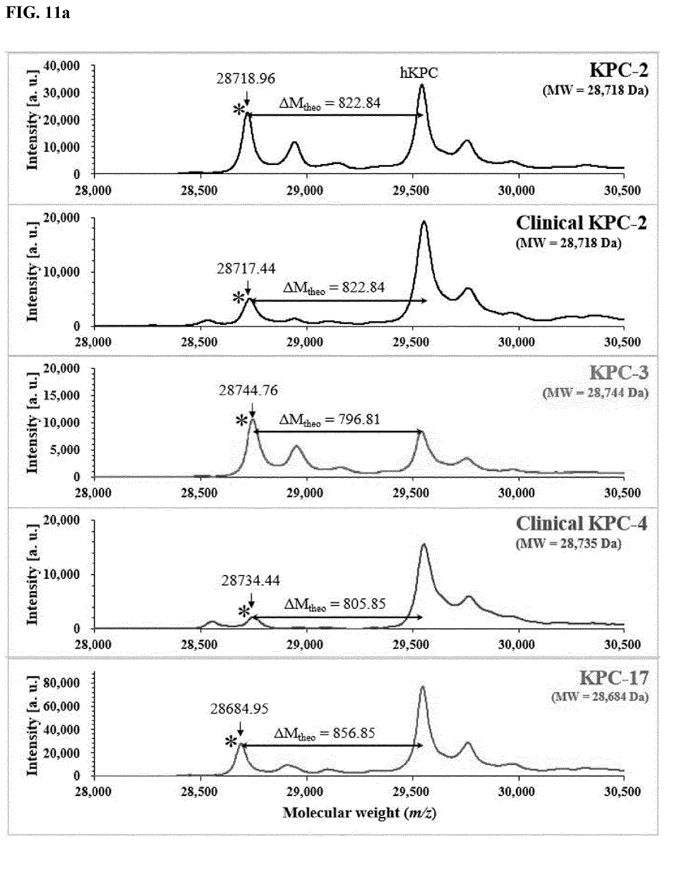

Analysis using Ex-Cal (KPC-2) and In-Cal (hKPC-2) was performed to identify the KPC subtype proteins (KPC-2, KPC-3, KPC-4, and KPC-17). MALDI mass spectra and average mass errors (-0.9 to 0.9 Da) for these proteins are shown in Table 1 and FIG. 11. All KPC subtypes were correctly identified with less than 1 Da error, regardless of whether they were standard or clinical strains, despite having mass differences ranging from -34 to +26 Da (Table 1 and FIG. 11b).

**[Table 1]**

| Source | Strains | Targets | Δmass^{a} (Da) | Av. mass error^{b} (Da) | SD^{c} (Da) |
|---|---|---|---|---|---|
| Standard | *E. coli* | KPC-2 | 0.0 | 0.9 | 0.6 |
| Clinical | *K. pn* | KPC-2 | 0.0 | -0.7 | 0.6 |
| Standard | *E. coli* | KPC-3 | -26.0 | 0.6 | 0.7 |
| Clinical | *K. pn* | KPC-4 | -17.0 | -0.7 | 0.3 |
| Standard | *E. coli* | KPC-17 | 34.0 | 0.9 | 0.1 |
| Average | | | | 0.76 | 0.32 |

### Determining the Mass Calibration Range Using Internal Standard Fluorine

To evaluate whether the method of the present invention using 6 x HIS-tagged protein as an internal standard material can be generally applied in other mass ranges, the present inventors checked the mass error of another target protein, ALDC, using hALDC. As an internal standard, hALDC tagged with 6 x His has a mass of 40.6 kDa, more than 12 kDa higher than hKPC-2. The present inventors performed an internal calibration (In-Cal) using six proteins (Ubi, Myo, Try, KPC-2, BSA, and hALDC) as candidate internal standards (FIG. 12a). To evaluate how the mass distance between the internal standard and the target protein affects the mass error, the ALDC mass error values after In-Cal were calculated. The mass distance of the six internal standard candidate proteins to ALDC ranged from approximately 2100 to 31000 Da (FIG. 12). The results showed that hALDC as an internal standard for the ALDC protein again yielded very low mass errors (<3.4 Da, <85 ppm), as was the case for hKPC-2. However, other internal standard candidates that differed by more than 10 kDa from the target protein did not function as standards at all (>3000 ppm error).

Having described specific embodiment of the present invention in detail above, it is to be understood that variants and modifications thereof falling within the spirit of the invention may become apparent to those skilled in this art, and the scope of this invention is to be determined by appended claims and their equivalents.

## Claims

1. An internal standard material for mass determination of a protein, comprising:
(a) the protein to be analyzed; and
(b) a tag peptide comprising 1 to 100 consecutive random amino acids bound to the N-terminus or C-terminus of the protein to be analyzed.

2. The internal standard material of claim 1, wherein said tag peptide comprises 2 to 80 consecutive random amino acids bound to the C-terminus of the protein be analyzed.

3. The internal standard material of claim 2, wherein the tag peptide is selected from the group consisting of a repeat sequence of the same amino acid residues; a consecutive sequence of different amino acid residues; a repeat of a consecutive sequence of different amino acid residues; and combinations thereof.

4. The internal standard material of claim 3, wherein the same amino acid is histidine (His).

5. The internal standard material of claim 3, wherein the consecutive sequence of different amino acid residues comprises an amino acid of SEQ ID NO: 7.

6. The internal standard material of claim 3, wherein the mass spectrometry is selected from the group consisting of matrix-assisted laser desorption/ ionization time-of-flight (MALDI-TOF) mass spectrometry, surface enhanced laser desorption/ ionization time-of-flight (SELDI-TOF) mass spectrometry, electrospray ionisation time-of-flight (ESI-TOF) mass spectrometry, liquid chromatography-mass spectrometry (LC-MS) and liquid chromatography-mass spectrometry/ mass spectrometry (LC-MS/MS).

7. The internal standard material of claim 6, wherein the mass spectrometry is a MALDI-TOF (Matrix Desorption/ Ionization Time of Flight) mass spectrometry.

8. A nucleic acid molecule encoding the internal standard material of any one of claims 1 to 7.

9. A method for detecting a protein in a biological sample comprising:
(a) adding the internal standard material of any one of claims 1 to 7 to the biological sample comprising the protein to be analyzed;
(b) measuring a mass value of the protein to be analyzed and the internal standard material in the biological sample; and
(c) calibrating the measured mass value of the protein to be analyzed based on the measured mass value of the internal standard material.

10. The method of claim 9, wherein the step (b) is performed using a mass spectrometry method selected from the group consisting of matrix-assisted laser desorption/ ionization time-of-flight (MALDI-TOF) mass spectrometry, surface enhanced laser desorption/ ionization time-of-flight (SELDI-TOF) mass spectrometry, electrospray ionisation time-of-flight (ESI-TOF) mass spectrometry, liquid chromatography-mass spectrometry (LC-MS) and liquid chromatography-mass spectrometry/ mass spectrometry (LC-MS/MS).

11. The method of claim 10, wherein the step (b) is performed using Matrix Desorption/ Ionization Time of Flight (MALDI-TOF) mass spectrometry.

12. An internal standard material for quantitation of a protein, comprising:
(a) the protein to be analyzed; and
(b) a tag peptide comprising 1 to 100 consecutive random amino acids bound to the N-terminus or C-terminus of the protein to be analyzed.

13. A method for quantitation of a protein in a biological sample comprising:
(a) adding the internal standard material of claim 12 to the biological sample comprising the protein to be analyzed;
(b) measuring a mass value of the protein to be analyzed and the internal standard material in the biological sample; and
(c) calibrating a quantitative value of the protein to be analyzed based on the signal intensity of the measured mass value of the internal standard material.

14. An internal standard material for mass determination of a protein comprising:
(a) any protein whose mass value is known; and
(b) a tag peptide comprising 1 to 100 consecutive random amino acids bound to the N-terminus or C-terminus of the protein.

15. A method for detecting a protein in a biological sample comprising:
(a) adding the internal standard material of claim 14 to the biological sample comprising the protein to be analyzed;
(b) measuring a mass value of the protein to be analyzed and the internal standard material in the biological sample; and
(c) calibrating the measured mass value of the protein to be analyzed based on the measured mass value of the internal standard material.

16. An internal standard material for quantitation of a protein comprising:
(a) any protein whose mass value is known; and
(b) a tag peptide comprising 1 to 100 consecutive random amino acids bound to the N-terminus or C-terminus of the protein.

17. A method for quantitation of a protein in a biological sample comprising:
(a) adding the internal standard material of claim 16 to the biological sample comprising the protein to be analyzed;
(b) measuring a mass value of the protein to be analyzed and the internal standard material in the biological sample; and
(c) calibrating a quantitative value of the protein to be analyzed based on the signal intensity of the measured mass value of the internal standard material.
